# EUROPEAN PATENT APPLICATION

(11) **EP 1 602 373 A1**
(43) Date of publication of application: **07.12.2005**
(21) Application number: 04714491.0
(22) Date of filing: 25.02.2004
(51) Int. Cl.: A61K 31/454, A61K 31/496, A61K 31/506, A61P 27/06, C07D 403/12

(54) **OCULAR HYPOTENSIVE AGENT**

(30) Priority: 28.02.2003 JP 2003053708
(71) Applicant: SUMITOMO PHARMACEUTICALS COMPANY, LIMITED, Osaka 541-8510 (JP)
(72) Inventor: MATSUSHIMA, Hiroaki, Rohto Pharmaceutical Co., Ltd, Osaka-shi, Osaka 544-8666 (JP); FUJINAGA, Kumiko, Rohto Pharmaceutical Co., Ltd., Osaka-shi, Osaka 544-8666 (JP)
(74) Representative: Vossius & Partner
(86) International application number: PCT/JP2004/002235
(87) International publication number: WO 2004/075895

(57) **Abstract**

It is intended to provide an ocular hypotensive agent serving as a prophylactic or therapeutic agent for glaucoma, ocular hypertension, etc. which contains a compound of the formula (1): 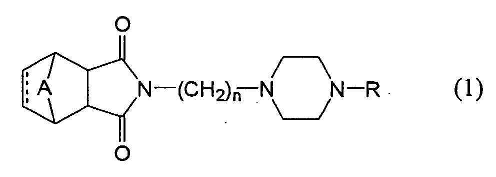 wherein A is methylene group, ethylene group or oxygen atom, n is an integer of 3 or 4, R is phenyl group optionally substituted, 2-pyridyl group optionally substituted or 2-pyrimidinyl group optionally substituted, and a solid line with a dotted line in the bicyclo ring means a single bond or a double bond.

## Description

### TECHNICAL FIELD

The present invention relates to an ocular hypotensive agent useful as, for instance, a prophylactic or therapeutic agent for glaucoma or ocular hypertension.

### BACKGROUND ART

It is described in Japanese Patent Publication A 58-126865 that an imide compound represented by the formula: 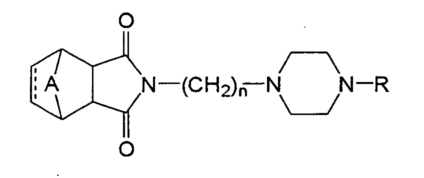 wherein R, A and n are the same as defined below, have anxiolytic activity and a compound among these compounds of the above formula, tandospirone citrate: 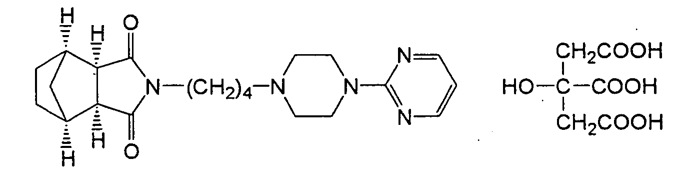 is commercialized in Japan as a therapeutic agent for neurosis or psychosomatic disease.

It is described in Japanese Patent Publication A 62-123179 that compounds represented by the formula: 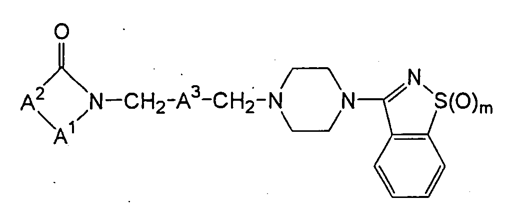 wherein A¹, A², A³ and m are the same as defined below, are useful as an antipsychotic, and a compound among these compounds of the above formula, perospirone hydrochloride hydrate: 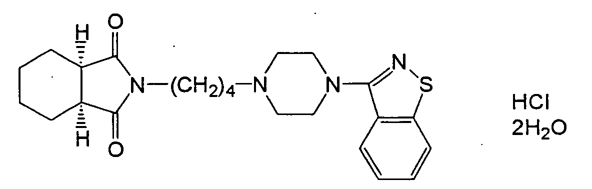 is commercialized in Japan as a therapeutic agent for schizophrenia.

Furthermore, it is described in Japanese Patent Publication A 1-199967 that compounds represented by the formula: 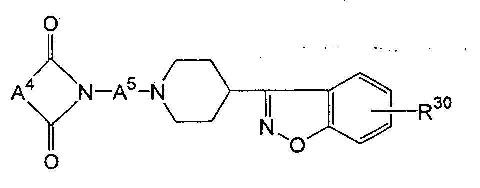 wherein A⁴, A⁵ and R³⁰ are the same as defined below, are useful as an antipsychotic, and a compound among these compounds of the above formula, N-[4-[4-(6-fluoro-1,2-benzisoxazol-3-yl)piperidin-1-yl]butyl]bicyclo[2.2.1]heptan-2,3-diexo-carboxyimide hydrochloride: 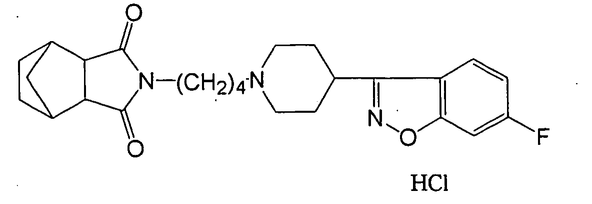 is described therein.

It is described in Japanese Patent Publication A 9-316002 that a certain 5-HT_{1A} ligand has ocular tension-reducing activity and is expected as a therapeutic agent for oculopathy such as glaucoma, etc. Furthermore a certain 5-HT_{1A} agonist or 5-HT₂ antagonist was reported to have ocular tension-reducing activity (Eye 14, 454-463, 2000).

### DISCLOSURE OF INVENTION

The present invention relates to an ocular hypotensive agent, more in detail, a prophylactic or therapeutic agent for glaucoma and ocular hypertension.

The present invention relates to an ocular hypotensive agent, more in detail, a prophylactic or therapeutic agent for glaucoma and ocular hypertension containing one of the following compounds or their pharmaceutically acceptable salts as an active ingredient:
the compound represented by the formula (1): 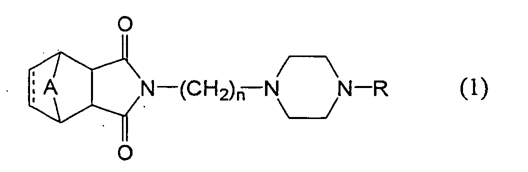 wherein A is methylene group, ethylene group or oxygen atom, n is an integer of 3 or 4, R is phenyl group optionally substituted, 2-pyridyl group optionally substituted or 2-pyrimidinyl group optionally substituted, and a solid line with a dotted line in the bicyclo ring means a single bond or a double bond,
the compound of the formula (2): 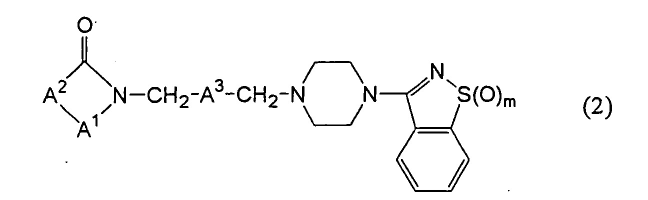 wherein A¹ is carbonyl group or sulfonyl group, and when A¹ is carbonyl group, A² is a group represented by the formula: 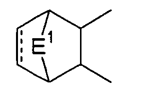 wherein E¹ is methylene group, ethylene group or oxygen atom, and a solid line with a dotted line is the same as defined above, the formula: 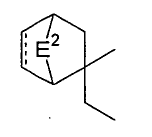 wherein E² is methylene group or ethylene group, and a solid line with a dotted line is the same as defined above, or the formula: 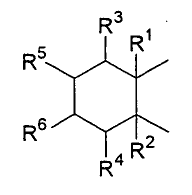 wherein R¹, R², R³, R⁴, R⁵ and R⁶ are independently hydrogen atom or methyl group,
and when A¹ is sulfonyl group, A² is 1,2-phenylene group, A³ is ethylene group optionally substituted by hydroxy group, ethenylene group or ethynylene and m is an integer of 0, 1 or 2,
or the compound of the formula (3): 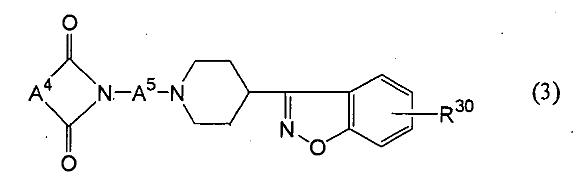 wherein R³⁰ is hydrogen atom, halogen atom, lower alkyl group, lower alkoxy group or hydroxy group, A⁴ is a group represented by the formula: 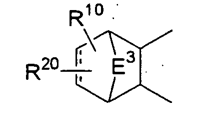 wherein R¹⁰ and R²⁰ are both hydrogen atom, or either of them is hydrogen atom and the other is hydroxy group, lower alkyl group or lower alkanoyloxy group, or R¹⁰ and R²⁰ are taken together to form an oxo group, E³ is methylene group, ethylene group or oxygen atom and a solid line with a dotted line is the same as defined above,
the formula: 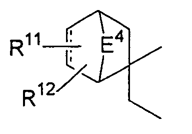 wherein E⁴ is methylene group or ethylene group, R¹¹ and R¹² are both hydrogen atom or either is hydrogen atom, and the other is hydroxy group, lower alkyl group or lower alkanoyloxy group, or R¹¹ and R¹² are taken together to form an oxo group, and a solid line with a dotted line is the same as defined above,
or the formula: 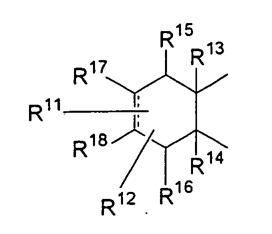 wherein R¹³, R¹⁴, R¹⁵, R¹⁶, R¹⁷ and R¹⁸ are independently hydrogen atom or lower alkyl group, and R¹¹, R¹² and a solid line with a dotted line are the same as defined above,
and A⁵ is lower alkylene group, lower alkenylene group or lower alkylene group substituted by hydroxy group.

Examples of the substituent of phenyl group in R of the formula (1) are halogen atom, C1-4 alkyl group, C1-4 alkoxy group, etc.

The term "lower alkyl" in the formula (3) means alkyl group having less than 8 carbon atoms, especially less than 6 carbon atoms, and these alkyl chains may be straight or branched. Examples of lower alkyl group are C1-7 alkyl group such as methyl, ethyl, propyl, isopropyl, butyl, etc. Examples of lower alkanoyloxy group are C2-7 alkanoyloxy group such as acetoxy, propanoyloxy, etc. Examples of lower alkylene group are C1-7 alkylene group such as trimethylene, tetramethylene, etc. Examples of lower alkenylene group are C2-7 alkenylene group such as propenylene, 2-butenylene, etc. Examples of lower alkoxy group are C1-7 alkoxy group such as methoxy, ethoxy, propoxy, isopropoxy, etc. Examples of halogen atom are fluorine atom, chlorine atom, bromine atom, etc.

Examples of a pharmaceutical acceptable salt of the compounds of the formulas (1), (2) and (3) are a salt with an inorganic acid such as hydrochloric acid, hydrobromic acid, hydroiodic acid, sulfuric acid, nitric acid, phosphoric acid, etc., and a salt with an organic acid such as citric acid, maleic acid, fumaric acid, tartaric acid, formic acid, acetic acid, trifluoroacetic acid, oxalic acid, methanesulfonic acid, benzenesulfonic acid, toluenesulfonic acid, etc.

Examples of an active ingredient in the preparation of the present invention are tandospirone citrate in the compounds of the formula (1), perospirone hydrochloride hydrate in the compounds of the formula (2) and N-[4-[4-(6-fluoro-1,2-benzisoxazol-3-yl)piperidin-1-yl]butyl]bicyclo[2.2.1]heptan-2,3-diexo-carboxyimide hydrochloride in the compounds of the formula (3). These compounds are preferable in view of duration for the activity.

The compounds of the formulas (1), (2) and (3) can be prepared in accordance with the methods respectively disclosed in Japanese Patent Publication A 58-126865, Japanese Patent Publication A 62-123179 and Japanese Patent Publication A 1-199967.

Glaucoma directed to the present invention widely means diseases caused by disturbance of ocular tissue, especially disturbance of function of optic nerve and disturbance of vision owing to promotion of ocular tension in regardless of pathogenesis. In regard to glaucoma, there are high tension glaucoma, and normal tension glaucoma which shows glaucoma papilla defect and defect of field of vision during showing normal tension, and in either case the therapy to reduce ocular tension is practiced.

Furthermore, ocular hypertension directed to the present invention is a disease that shows high ocular tension beyond normal ocular tension, but does not show any disturbance in visual function and there is high possibility to progress into glaucoma after long term. In ocular hypertension, an ocular tension-reducing agent is administered in earlier stage to protect the progress to glaucoma.

When the compounds directed to the present invention is administered orally or parenterally, as the reducing effect of ocular tension is shown, the compounds are useful as a prophylactic or therapeutic agent for glaucoma and ocular hypertension.

The preparation of the present invention is orally or parenterally administered. The preparation is administered in the conventional form, namely orally in tablets, capsules, granules, powders, etc. or in the form of solutions, emulsions, suspensions, etc. as injections or eye drops. The preparation is administered in ophthalmic ointments, or in creams, solutions or patches as a dermal application agent. The preparation can be administered to rectum in the form of suppositories. These preparations are prepared by adding to an active ingredient an acceptable carrier, filler, binder, stabilizer, buffer, solubilizing agent, osmotic agent, etc. in accordance with the conventional method.

The dose or administration times vary depending on conditions of disease, age, body weight, administration form, but in case of oral administration of tandospirone citrate, usually the dose is 10 to 100mg/day/adult, preferably 20 to 60mg/day/adult in a single dose or divided doses.

In case of administration of the preparation of the present invention by instillation or by intraocular, the active ingredient is dissolved in a physiological saline or physiologically acceptable buffer so as to be 0.01 to 1% in the concentration, and the solution is used. For example, in case of eye drops, usually, the preparation is administered 0.5 to 500mg/day/adult, preferably, 1 to 100mg/day/adult in a single dose or divided doses.

### Example

The present invention is explained by the following examples in detail, but the present invention should not be limited by these examples.

### Example 1 Ocular tension-reducing activity by administration to anterior chamber

Test solutions 1 to 3 were prepared as mentioned below. The solutions were administered in anterior chamber of the rabbit according to the test method mentioned below, and their ocular tension-reducing activities comparing with normal ocular tension of the rabbit were confirmed.

### [Method for preparing test solution]

Tandospirone citrate or perospirone hydrochloride was dissolved in a physiological saline to prepare following preparations.
Test solution 1: 0.1% tandospirone citrate solution
Test solution 2: 0.1% perospirone hydrochloride hydrate solution
Test solution 3: 0.03% perospirone hydrochloride hydrate solution

### [Test method]

Non-white 5 male rabbits were used. Ocular tension was measured under topically anesthetizing the corneal surface with 0.4% oxybuprocaine hydrochloride by using an air impression electronic tonometer (by Japan Alcon Company).

The rabbit was fitted in a cylinder, after topically anesthetizing the corneal surface with 0.4% oxybuprocaine hydrochloride, and the test solution 10µl was injected via cornea in anterior chamber of one eye with an injection needle (30G). To the other eye as a control eye, in the same way as mentioned above, a physiological saline was injected. Ocular tension just before and after injection of the test solution or the physiological saline was measured in accordance with the same method as mentioned above. At each measured time, the difference (mean ± S.E.) between tension in the eye to which the test solution was administered and tension in the control eye, and the corresponding t-test result was calculated. The result was shown in Table 1.

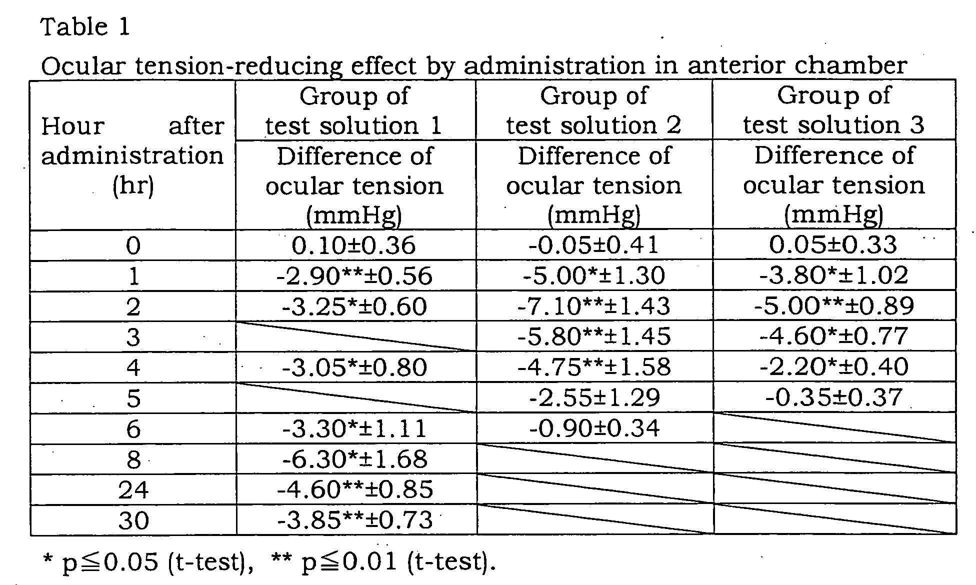

From the above result, in the groups to which the test solution was administered, the significant ocular tension-reduction was observed from one hour after administration in anterior chamber. In regard to the test solution 1, its effect was continued for more than 30 hours, and the maximum ocular tension-reducing rate was 6.3mmHg 8 hours after administration. In regard to the test solution 2, its effect was continued for 4 hours with the maximum ocular tension-reducing rate about 5.0mmHg. In regard to the test solution 3, its effect was continued for 4 hours with the maximum ocular tension-reducing rate about 7.1 mmHg.

### Example 2 Ocular tension-reducing activity by instillation

Test solutions 4 and 5 were prepared as mentioned below and their ocular tension-reducing activities comparing with normal ocular tension of the rabbit was confirmed by installation. The method for measuring ocular tension was carried out in accordance with the method mentioned in Example 1.

### [Method for preparing test solution]

Test solution 4 was prepared by dissolving perospirone hydrochloride in solution 1 (physiological saline containing 0.5% polysolbate 80), and test solution 5 was prepared by dissolving perospirone hydrochloride in solution 2 (physiological saline containing 0.5% polysolbate 80, 0.5% glycerin, 2% citric acid and 7% polyethylene glycol 4000, pH4.1). The test solutions were adjusted to each concentration.
Test solution 4: 0.3% perospirone hydrochloride hydrate solution
Test solution 5: 1.0% perospirone hydrochloride hydrate solution

### [Test method]

Test solutions 4 and 5 were instillated to one eye of the non-white rabbit. Solution 1 (50µl) as a control of the test solution 4 and solution 2 (50µl) as a control of the test solution 5 were instillated to the other eye. Ocular tension just before and after injection .of the test solution and the physiological saline were measured in accordance with the same method as mentioned above. At each measured time, the difference (mean ± S.E.) between tension in the eye to which the test solution was administered and tension in the control eye, and the corresponding t-test result was calculated. The result was shown in Table 2.

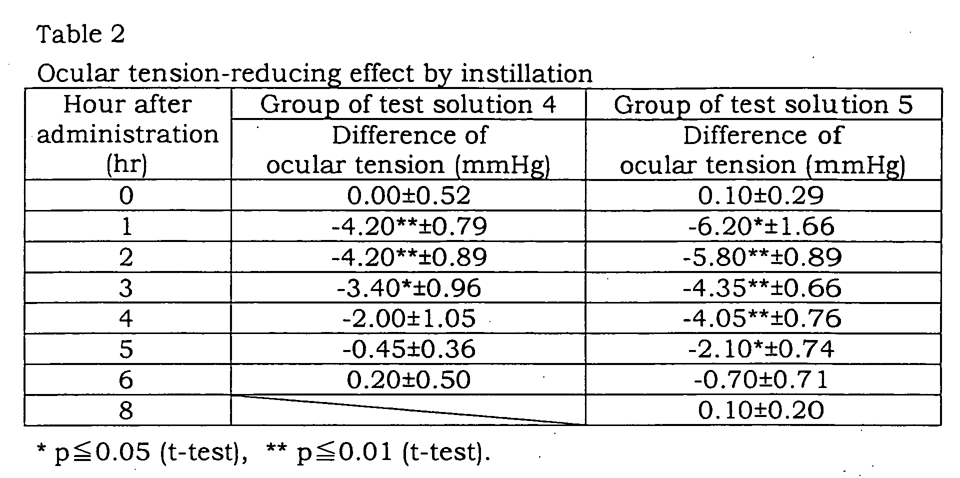

From the above result, in the groups to which the test solution was administered, the significant ocular tension-reduction was observed from one hour after administration in anterior chamber.

### Example 3 Ocular tension-reducing activity by administration in anterior chamber of N-[4-[4-(6-fluoro-1,2-benzisoxazol-3-yl)piperidin-1-yl]butyl]bicyclo[2.2.1]heptan-2,3-diexo-carboxyimide hydrochloride (Abbreviated as compound A)

Test solution 6 was prepared as mentioned below and its ocular tension-reducing activity comparing with normal ocular tension of the rabbits was confirmed by instillation.

### [Method for preparing test solution]

Following solution was prepared by dissolving compound A in physiological saline.

Test solution 6: 0.03% compound A solution

### [Test method]

Ocular tension just before and after injection of the test solution and the physiological saline was measured in accordance with the same method as mentioned in Example 1. At each measured time, the difference (mean ± S.E.) between tension in the eye to which the test solution was administered and tension in the control eye, and the corresponding t-test result was calculated. The result was shown in Table 3.

**Table 3**

| Ocular tension-reducing effect by administration in anterior chamber | |
|---|---|
| Hour after administration (hr) | Group of test solution 6 |
| | Difference of ocular tension (mmHg) |
| 0 | 0.00±0.11 |
| 1 | -3.05±1.44 |
| 2 | -3.35*±0.97 |
| 3 | -2.50±0.91 |
| 4 | -2.10±0.91 |
| 5 | -0.35±0.68 |

| | |
|---|---|
| * p≦0.05 (t-test), ** p ≦ 0.01 (t-test). | |

From the above result, in the group to which the test solution 6 was administered, the significant maximum ocular tension-reduction was observed two hours after administration in anterior chamber.

### Example 4 Ocular tension-reducing activity by instillation of compound A

Test solution 7 was prepared as mentioned below and its ocular tension-reducing activity comparing with normal ocular tension of the rabbit was confirmed by instillation. The measuring method of ocular tension was carried out in accordance with the method mentioned in Example 1.

### [Method for preparing test solution]

Following solution was prepared by dissolving compound A in solution 3 (physiological saline containing 0.5% polysolbate 80).
Test solution 7: 0.1% compound A solution

### [Test method]

Ocular tension just before and after the injection of the test solution and the solution 3 was measured in accordance with the same method as mentioned in Example 2. At each measured time, the difference (mean ± S.E.) between tension in the eye to which the test solution was administered and tension in the control eye, and the corresponding t-test result was calculated. The result was shown in Table 4.

**Table 4**

| Ocular tension-reducing effect by instillation | |
|---|---|
| Hour after administration (hr) | Group of test solution 7 |
| | Difference of ocular tension (mmHg) |
| 0 | 0.15±0.23 |
| 1 | -4.10**±0.64 |
| 2 | -3.70*± 1.09 |
| 3 | -1.00±0.64 |
| 4 | -0.60±0.51 |

| | |
|---|---|
| * p≦0.05 (t-test), | |
| ** p≦0.01 (t-test). | |

From the above result, in the group to which the test solution 7 was administered, it was observed that the ocular tension-reduction was continued for 2 hours with the maximum ocular tension-reduction about 4.1mmHg.

As tandospirone citrate, perospirone hydrochloride hydrate and compound A have ocular tension-reducing activity, they are useful as an ocular hypotensive agent, more concretely a prophylactic or therapeutic agent for glaucoma and ocular hypertension.

| Preparation 1 | |
|---|---|
| Eye drop (Ingredients 1 in 100ml) | |
| Perospirone hydrochloride hydrate | 1.0g |
| Polysolbate 80 | 0.5g |
| Polyethylene glycol 4000 | 5.0g |
| Citric acid | 2.0g |
| Sodium chloride | 0.4g |
| Sodium hydroxide | suitable amount |
| Sterilized purified water | suitable amount |
| Total | 100 ml |

According to above ingredients, to sterilized distilled water 80ml were added perospirone hydrochloride hydrate, polysorbate 80, polyethylene glycol 4000, citric acid and sodium chloride to dissolved, and then, the solution was adjusted with sodium hydroxide to pH4.1. Thereto was added sterilized distilled water to make eye drop having total amount of 100ml.

| Preparation 2 | |
|---|---|
| Eye drop (Ingredients 2 in 100ml) | |
| Tandospirone citrate | 0.1g |
| Polysolbate 80 | 0.5g |
| Boric acid | 1.0g |
| Sodium chloride | 0.4g |
| Sodium hydroxide | suitable amount |
| Sterilized distilled water | suitable amount |
| Total | 100 ml |

According to above ingredients 2, to sterilized distilled water 80ml were added tandospirone citrate, polysorbate 80, boric acid and sodium chloride to dissolved, and then, the solution was adjusted with sodium hydroxide to pH6.0. Thereto was added sterilized distilled water to make eye drop having total amount of 100ml.

### INDUSTRIAL APPLICABILITY

The present invention can provide an ocular hypotensive agent. The present invention in more detail provides a prophylactic and therapeutic agent for glaucoma and ocular hypertension.

## Claims

1. An ocular hypotensive agent containing one of the following compounds or their pharmaceutically acceptable salts as an active ingredient:
a compound represented by the formula (1): 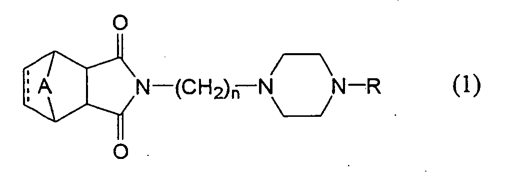 wherein A is methylene group, ethylene group or oxygen atom, n is an integer of 3 or 4, R is phenyl group optionally substituted, 2-pyridyl group optionally substituted or 2-pyrimidinyl group optionally substituted, and a solid line with a dotted line in the bicyclo ring means a single bond or a double bond,
a compound of the formula (2): 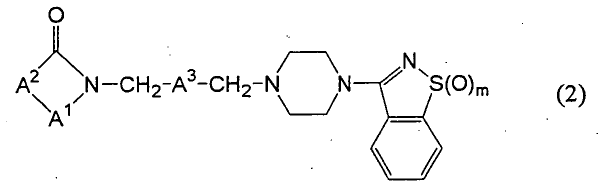 wherein A¹ is carbonyl group or sulfonyl group, and when A¹ is carbonyl group, A² is a group represented by the formula: 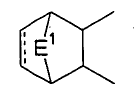 wherein E¹ is methylene group, ethylene group or oxygen atom, and a solid line with a dotted line is the same as defined above,
the formula: 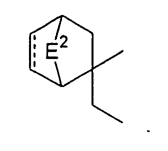 wherein E² is methylene group or ethylene group, and a solid line with a dotted line is the same as defined above,
or the formula: 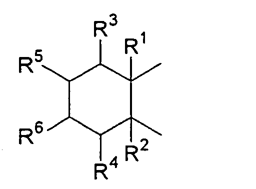 wherein R¹, R², R³, R⁴, R⁵ and R⁶ are independently hydrogen atom or methyl group,
and when A¹ is sulfonyl group, A² is 1,2-phenylene group, A³ is ethylene group optionally substituted by hydroxy group, ethenylene group or ethynylene and m is an integer of 0, 1 or 2,
or a compound of the formula (3): 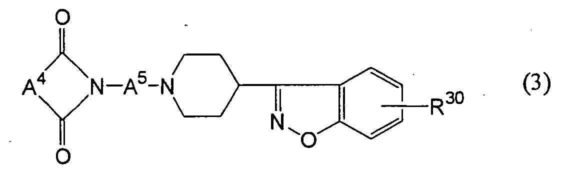 wherein R³⁰ is hydrogen atom, halogen atom, lower alkyl group, lower alkoxy group or hydroxy group, A⁴ is a group represented by the formula: 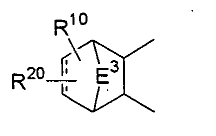 wherein R¹⁰ and R²⁰ are both hydrogen atom, or either of them is hydrogen atom and the other is hydroxy group, lower alkyl group or lower alkanoyloxy group, or R¹⁰ and R²⁰ are taken together to form an oxo group, E³ is methylene group, ethylene group or oxygen atom and a solid line with a dotted line is the same as defined above,
the formula: 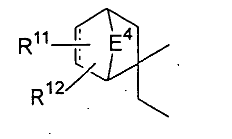 wherein E⁴ is methylene group or ethylene group, R¹¹ and R¹² are both hydrogen atom or either is hydrogen atom, and the other is hydroxy group, lower alkyl group or lower alkanoyloxy group, or R¹¹ and R¹² are taken together to form an oxo group, and a solid line with a dotted line is the same as defined above,
or the formula: 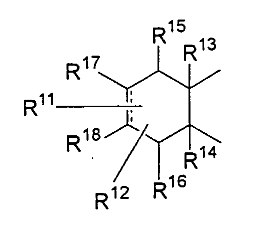 wherein R¹³, R¹⁴, R¹⁵, R¹⁶, R¹⁷ and R¹⁸ are independently hydrogen atom or lower alkyl group, and R¹¹, R¹² and a solid line with a dotted line are the same as defined above,
and A⁵ is lower alkylene group, lower alkenylene group or lower alkylene group substituted by hydroxy group.

2. The ocular hypotensive agent according to claim 1 containing tandospirone, perospirone or N-[4-[4-(6-fluoro-1,2-benzisoxazol-3-yl) piperidin-1-yl]butyl]bicyclo[2.2.1]heptan-2,3-diexo-carboxyimide or its pharmaceutically acceptable salt.

3. The ocular hypotensive agent according to claim 1 containing tandospirone citrate, perospirone hydrochloride or N-[4-[4-(6-fluoro-1,2-benzisoxazol-3-yl)piperidin-1-yl]butyl]bicyclo[2.2.1]heptan-2,3-diexocarboxyimide hydrochloride.

4. A prophylactic or therapeutic agent for glaucoma or ocular hypertension containing as an active ingredient the compound or its pharmaceutically acceptable salt described in claim 1, 2, or 3.
